# EUROPEAN PATENT SPECIFICATION

(11) **EP 3 391 453 B1**
(45) Date of publication and mention of the grant of the patent: **08.07.2020**
(21) Application number: 16808671.8
(22) Date of filing: 08.12.2016
(51) Int. Cl.: H01M 10/0567, C07C 309/84, H01M 10/0525, H01M 10/0568, H01M 10/0569

(54) **CYANOALKYL SULFONYLFLUORIDES FOR ELECTROLYTE COMPOSITIONS FOR HIGH ENERGY LITHIUM-ION BATTERIES**
CYANALKYL-SULFONYLFLUORIDE FÜR ELEKTROLYTZUSAMMENSETZUNGEN FÜR ENERGIEREICHE LITHIUM-IONEN-BATTERIEN
CYANOALKYLE DE FLUORURE DE SULFONYLE POUR DES COMPOSITIONS D'ÉLECTROLYTE DE BATTERIES AU LITHIUM À HAUTE ÉNERGIE

(30) Priority: 17.12.2015 EP 15200845
(43) Date of publication of application: 24.10.2018
(73) Proprietor: BASF SE, 67056 Ludwigshafen am Rhein (DE)
(72) Inventor: HAN, Zhenji, Osaka Osaka 554-0002 (JP); FUKUZUMI, Takeo, Toyonaka-shi 560-0082 (JP); SCHULZ-DOBRICK, Martin, Osaka 553-0003 (JP)
(74) Representative: BASF IP Association
(86) International application number: PCT/EP2016/080318
(87) International publication number: WO 2017/102557

(56) References cited:
- EP-A1- 2 750 238
- JP-A- 2009 054 288
- ZHANG ET AL: "A review on electrolyte additives for lithium-ion batteries", JOURNAL OF POWER SOURCES, ELSEVIER SA, CH, vol. 162, no. 2, 22 November 2006 (2006-11-22), pages 1379-1394, XP027938606, ISSN: 0378-7753 [retrieved on 2006-11-22]

## Description

The present invention relates to compounds of formula (I) 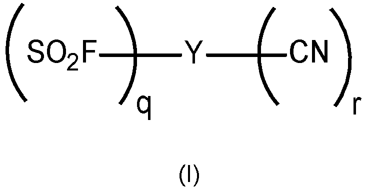 wherein
q is 1, 2 or 3,
r is 1, 2 or 3, and
Y is a q+r valent alkylene group having 1 to 12 C-atoms, wherein one or more CH₂-units of the alkylene group which are not directly bound to CN or SO₂F may be replaced by O;
and their use in electrolyte compositions for electrochemical cells, electrolyte compositions containing one or more compounds of formula (I) and electrochemical cells comprising such electrolyte compositions.

Storing electrical energy is a subject of still growing interest. Efficient storage of electric energy allows electric energy to be generated when it is advantageous and to be used when needed. Secondary electrochemical cells are well suited for this purpose due to their reversible conversion of chemical energy into electrical energy and vice versa (rechargeability). Secondary lithium batteries are of special interest for energy storage since they provide high energy density and specific energy due to the small atomic weight of the lithium ion, and the high cell voltages that can be obtained (typically 3 to 5 V) in comparison with other battery systems. For that reason, these systems have become widely used as a power source for many portable electronics such as cellular phones, laptop computers, mini-cameras, etc.

In secondary lithium batteries like lithium ion batteries organic carbonates, ethers, esters and ionic liquids are used as sufficiently polar solvents for solvating the conducting salt(s). Most state of the art lithium ion batteries in general comprise not a single solvent but a solvent mixture of different organic aprotic solvents.

Besides solvent(s) and conducting salt(s) an electrolyte composition usually contains further additives to improve certain properties of the electrolyte composition and of the electrochemical cell comprising said electrolyte composition. Common additives are for example flame retardants, overcharge protection additives and film forming additives which react during first charge/discharge cycle on the electrode surface thereby forming a film on the electrode. Organic sulfonyl fluorides are a class of additives for electrolyte compositions which are used to improve the high temperature performance of lithium ion batteries.

US 9,136,560 B2 discloses electrolyte compositions containing alkyl and alkenyl monosulfonyl fluorides to inhibit generation of gas during cycling of a secondary lithium battery and to improve high-temperature-storage characteristics.

US 2009/0053612 A1 describes the use of alkyl di- and oligosulfonylfluorides or unsaturated monosulfonylfluorides in electrolyte compositions for improving the storage, cycling and swelling characteristics.

EP 2 750 238 A1 refers to non-aqueous electrolytes and batteries containing fluorinated and non-fluorinated alkyl sulfonylfluorides which additionally have a carboxylic acid derivative group. The carboxylic acid derivative group carrying sulfonylfluorides are added to improve the durability at high temperatures of batteries.

WO 2014/157591 A1 describes secondary batteries comprising an electrolyte composition containing different organic sulfonylfluorides with organic groups selected from fluorinated and non-fluorinated alkyl, alkenyl, phenyl, benzyl and the like.

Nevertheless there is still the need for further additives for electrolyte compositions which improve the performance of electrochemical cells and can be produced economically.

It is an objective of the present invention to provide further additives for electrolyte compositions improving the performance and electrochemical characteristics of electrochemical cells comprising such electrolyte composition like cycle stability, energy density, power capability and a long shelf life, in particular the performance at elevated temperatures. The additives should be producible economically.

Accordingly, the compounds of formula (I) defined at the outset, their use in electrolyte compositions, electrolyte compositions containing at least one compound of formula (I) and electrochemical cell comprising such electrolyte compositions are provided. The electrochemical cells comprising the electrolyte compositions exhibit good capacity retention during cycling at elevated temperatures, and high initial coulombic efficiency. Surprisingly the addition of a compound of formula (I) also reduces impedance built up in the cell. Since the alkylene group is non-fluorinated they can be produced cost efficient.

In the following the invention is described in detail.

One aspect of the invention as defined in claim 1 relates to electrolyte compositions comprising at least one compound of formula (I) 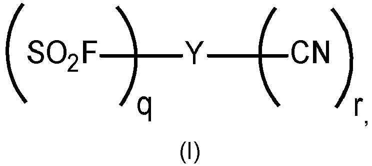 wherein
q is 1, 2 or 3,
r is 1, 2 or 3, and
Y is a q+r valent alkylene group having 2 to 6 C-atoms, wherein one or more CH₂-units of the alkylene group which are not directly bound to CN or SO₂F may be replaced by O. The q+r valent alkylene group having 2 to 6 C-atoms is not fluorinated, i.e. Y is a q+r valent non-fluorinated alkylene group having 2 to 6 C-atoms.
CN denotes the cyano group, SO₂F denotes the sulfonylfluoride group.
q is 1, 2, or 3, preferably q is 1 or 2.
r is 1, 2, or 3, preferably r is 1 or 2.
r and q may be equal or different, e.g.
r is 1 and q is 1,
r is 2 and q is 1,
r is 1 and q is 2,
r is 2 and q is 2,
r is 3 and q is 1,
r is 1 and q is 3,
r is 2 and q is 3,
r is 3 and q is 2, or
r is 3 and q is 3.

Preferably r is 1 and q is 1 or r is 2 and q is 2.

According to one embodiment q+r are at least 3.

The q+r valent alkylene group Y may be branched or linear, preferably Y is a linear alkylene group.

The alkylene group Y is derived from alkanes having 2 to 6 C-atoms. Examples of compounds from which the alkylene group Y may be derived are methane, ethane, n-propane, i-propane, n-butane, i-butane, n-pentane, n-hexane, and the like. The alkylene group Y is obtained from the respective alkane by replacing q hydrogens of the alkanes by SO₂F and replacing r hydrogens of the alkane by CN. Preferably the alkylene group Y is derived from ethane, n-propane, n-butane, n-pentane, and n-hexane.

The q+r valent alkylene group Y has 2 to 6 C-atoms, e.g. Y may have, 1, 2, 3, 4, 5 or 6 C-atoms

According to one embodiment r is 2 and the two sulfonylfluoride groups are bound to the alkylene group Y at terminal positions.

According to another embodiment q is 2 and both CN-groups are bound to the same C-atom of the alkylene group Y.

Preferred examples of compounds of formula (I) are 2-cyanoethanesulfonyl fluoride and 3,3-dicyano-pentane-1,5-disulfonyl fluoride.

Another aspect of the present invention as defined in claim 13 is the use of the compounds of formula (I) in electrolyte compositions, preferably in electrolyte compositions for electrochemical cells, e.g. in lithium ion capacitors, double layer capacitors and lithium batteries, in particular in secondary lithium batteries as described below. The compounds are can be used as additives for reducing the impedance build up in electrochemical cells, for reducing gas generation during the cycling and high temp storage, and for stabilizing the open circuit voltage. They can also be used as film forming additives.

The compounds of formula (I) are usually used in the electrolyte compositions by adding the desired amounts of the compound(s) of formula (I) to the electrolyte composition. They are usually used in the concentrations given below for the electrolyte compositions containing at least one compound of formula (I).

Viewed chemically, an electrolyte composition is any composition which comprises free ions and as a result is electrically conductive. The electrolyte composition functions as a medium that transfers ions participating in the electrochemical reaction taking place in an electrochemical cell. The most common electrolyte composition is an ionic solution, although molten electrolyte compositions and solid electrolyte compositions are likewise possible. An electrolyte composition of the invention is therefore an electrically conductive medium, primarily due to the presence of at least one substance which is present in a dissolved and/or molten state, i.e., an electrical conductivity supported by movement of ionic species. In liquid or gel electrolyte compositions the conducting salt is usually solvated in the aprotic organic solvent(s).

Another aspect of the invention refers to electrolyte compositions containing at least one compound of formula (I). The electrolyte composition may contain one compound of formula (I), it may contain more than one compound of formula (I), e.g. 2, 3 or more.

Usually the electrolyte composition contains in total at least 0.01 wt.-% of the at least one compound of formula (I), based on the total weight of electrolyte composition, preferably at least 0.02 wt.-%, and more preferred at least 0.2 wt.-%, based on the total weight of electrolyte composition. The maximum value of the total concentration of compounds of formula (I) in the electrolyte composition is usually 10 wt.-%, based on the total weight of electrolyte composition, preferably 5 wt.-%, and more preferred the upper limit of the total concentration of compounds of formula (I) is 3 wt.-%, based on the total weight of electrolyte composition. Usually the electrolyte composition contains in total 0.01 to 10 wt.-%, of the at least one compound of formula (I), based on the total weight of electrolyte composition, preferably 0.02 to 5 wt.-%, and more preferably 0.2 to 3 wt.-%.

Preferably the electrolyte composition contains at least one aprotic organic solvent. The at least one aprotic organic solvent may be selected from fluorinated and non-fluorinated aprotic organic solvents i.e. the electrolyte composition may contain a mixture of fluorinated and non-fluorinated aprotic organic solvents. The aprotic organic solvent is preferably selected from fluorinated and non-fluorinated cyclic and acyclic organic carbonates, di-C₁-C₁₀-alkylethers, di-C₁-C₄-alkyl-C₂-C₆-alkylene ethers and polyethers, cyclic ethers, cyclic and acyclic acetales and ketales, orthocarboxylic acids esters, cyclic and acyclic esters and diesters of carboxylic acids, cyclic and acyclic sulfones, and cyclic and acyclic nitriles and dinitriles and mixtures thereof. More preferred the aprotic organic solvent is selected from cyclic and acyclic organic carbonates, and most preferred, electrolyte composition contains at least two solvents selected from cyclic and acyclic organic carbonates, electrolyte composition contains at least one solvent selected from cyclic organic carbonates and at least one solvent selected from acyclic organic carbonates.

Examples of cyclic carbonates are ethylene carbonate (EC), propylene carbonate (PC) and butylene carbonate (BC), wherein one or more H in may be substituted by F and/or an C₁ to C₄ alkyl group, e.g. 4-methyl ethylene carbonate, monofluoroethylene carbonate (FEC), and cis- and trans-difluoroethylene carbonate. Preferred cyclic carbonates are ethylene carbonate, monofluoroethylene carbonate, and propylene carbonate, in particular ethylene carbonate.

Examples of acyclic carbonates are di-C₁-C₁₀-alkylcarbonates, wherein each alkyl group is selected independently from each other and wherein one or more H in may be substituted by F. Preferred are di-C₁-C₄-alkylcarbonates. Examples are e.g. diethyl carbonate (DEC), ethyl methyl carbonate (EMC), dimethyl carbonate (DMC), and methylpropyl carbonate. Preferred acyclic carbonates are diethyl carbonate (DEC), ethyl methyl carbonate (EMC), and dimethyl carbonate (DMC).

In one embodiment of the invention the electrolyte composition contains mixtures of acyclic organic carbonates and cyclic organic carbonates at a ratio by weight of from 1:10 to 10:1, preferred of from 3:1 to 1:1.

According to the invention each alkyl group of the di-C₁-C₁₀-alkylethers is selected independently from the other. Examples of di-C₁-C₁₀-alkylethers are dimethylether, ethylmethylether, diethylether, methylpropylether, diisopropylether, and di-n-butylether.

Examples of di-C₁-C₄-alkyl-C₂-C₆-alkylene ethers are 1,2-dimethoxyethane, 1,2-diethoxyethane, diglyme (diethylene glycol dimethyl ether), triglyme (triethyleneglycol dimethyl ether), tetraglyme (tetraethyleneglycol dimethyl ether), and diethylenglycoldiethylether.

Examples of suitable polyethers are polyalkylene glycols, preferably poly-C₁-C₄-alkylene glycols and especially polyethylene glycols. Polyethylene glycols may comprise up to 20 mol% of one or more C₁-C₄-alkylene glycols in copolymerized form. Polyalkylene glycols are preferably dimethyl- or diethyl- end-capped polyalkylene glycols. The molecular weight M_{w} of suitable polyalkylene glycols and especially of suitable polyethylene glycols may be at least 400 g/mol. The molecular weight M_{w} of suitable polyalkylene glycols and especially of suitable polyethylene glycols may be up to 5 000 000 g/mol, preferably up to 2 000 000 g/mol.

Examples of cyclic ethers are 1,4-dioxane, tetrahydrofuran, and their derivatives like 2-methyl tetrahydrofuran.

Examples of acyclic acetals are 1,1-dimethoxymethane and 1,1-diethoxymethane. Examples of cyclic acetals are 1,3-dioxane, 1,3-dioxolane, and their derivatives such as methyl dioxolane.

Examples of acyclic orthocarboxylic acid esters are tri-C₁-C₄ alkoxy methane, in particular trimethoxymethane and triethoxymethane. Examples of suitable cyclic orthocarboxylic acid esters are 1,4-dimethyl-3,5,8-trioxabicyclo[2.2.2]octane and 4-ethyl-1-methyl-3,5,8-trioxabicyclo[2.2.2]octane.

Examples of acyclic esters of carboxylic acids are ethyl and methyl formiate, ethyl and methyl acetate, ethyl and methyl proprionate, and ethyl and methyl butanoate, and esters of dicarboxylic acids like 1,3-dimethyl propanedioate. An example of a cyclic ester of carboxylic acids (lactones) is γ-butyrolactone.

Examples of cyclic and acyclic sulfones are ethyl methyl sulfone, dimethyl sulfone, and tetrahydrothiophene-S,S-dioxide (sulfolane).

Examples of cyclic and acyclic nitriles and dinitriles are adipodinitrile, acetonitrile, propionitrile, and butyronitrile.

The electrolyte composition usually contains at least one conducting salt. The electrolyte composition functions as a medium that transfers ions participating in the electrochemical reaction taking place in an electrochemical cell. The conducting salt(s) present in the electrolyte composition are usually solvated in the aprotic organic solvent(s). The conducting salt is preferably a lithium conducting salt. Examples of lithium conducting salts are
- Li[F₆₋ₓP(C_{y}F_{2y+1})ₓ], wherein x is an integer in the range from 0 to 6 and y is an integer in the range from 1 to 20;
   Li[B(R^{I})₄], Li[B(R^{I})₂(OR^{II}O)] and Li[B(OR^{II}O)₂] wherein each R^{I} is independently from each other selected from F, Cl, Br, I, C₁-C₄ alkyl, C₂-C₄ alkenyl, C₂-C₄ alkynyl, OC₁-C₄ alkyl, OC₂-C₄ alkenyl, and OC₂-C₄ alkynyl wherein alkyl, alkenyl, and alkynyl may be substituted by one or more OR^{III}, wherein R^{III} is selected from C₁-C₆ alkyl, C₂-C₆ alkenyl, and C₂-C₆ alkynyl, and
   (OR^{II}O) is a bivalent group derived from a 1,2- or 1,3-diol, a 1,2- or 1,3-dicarboxlic acid or a 1,2- or 1,3-hydroxycarboxylic acid, wherein the bivalent group forms a 5- or 6-membered cycle via the both oxygen atoms with the central B-atom;
- LiClO₄; LiAsF₆; LiCF₃SO₃; Li₂SiF₆; LiSbF₆; LiAlCl₄, Li(N(SO₂F)₂), lithium tetrafluoro (oxalato) phosphate; lithium oxalate; and
- salts of the general formula Li[Z(CₙF₂ₙ₊₁SO₂)ₘ], where m and n are defined as follows:
   m = 1 when Z is selected from oxygen and sulfur,
   m = 2 when Z is selected from nitrogen and phosphorus,
   m = 3 when Z is selected from carbon and silicon, and
   n is an integer in the range from 1 to 20.

Suited 1,2- and 1,3-diols from which the bivalent group (OR^{II}O) is derived may be aliphatic or aromatic and may be selected, e.g., from 1,2-dihydroxybenzene, propane-1,2-diol, butane-1,2-diol, propane-1,3-diol, butan-1,3-diol, cyclohexyl-trans-1,2-diol and naphthalene-2,3-diol which are optionally are substituted by one or more F and/or by at least one straight or branched non fluorinated, partly fluorinated or fully fluorinated C₁-C₄ alkyl group. An example for such 1,2- or 1,3-diole is 1,1,2,2-tetra(trifluoromethyl)-1,2-ethane diol.

"Fully fluorinated C₁-C₄ alkyl group" means, that all H-atoms of the alkyl group are substituted by F.

Suited 1,2- or 1,3-dicarboxlic acids from which the bivalent group (OR^{II}O) is derived may be aliphatic or aromatic, for example oxalic acid, malonic acid (propane-1,3-dicarboxylic acid), phthalic acid or isophthalic acid, preferred is oxalic acid. The 1,2- or 1,3-dicarboxlic acid are optionally substituted by one or more F and/or by at least one straight or branched non fluorinated, partly fluorinated or fully fluorinated C₁-C₄ alkyl group.

Suited 1,2- or 1,3-hydroxycarboxylic acids from which the bivalent group (OR^{II}O) is derived may be aliphatic or aromatic, for example salicylic acid, tetrahydro salicylic acid, malic acid, and 2-hydroxy acetic acid, which are optionally substituted by one or more F and/or by at least one straight or branched non fluorinated, partly fluorinated or fully fluorinated C₁-C₄ alkyl group. An example for such 1,2- or 1,3-hydroxycarboxylic acids is 2,2-bis(trifluoromethyl)-2-hydroxy-acetic acid.

Examples of Li[B(R^{I})₄], Li[B(R^{I})₂(OR^{II}O)] and Li[B(OR^{II}O)₂] are LiBF₄, lithium difluoro oxalato borate and lithium dioxalato borate.

Preferably the at least one conducting salt is selected from LiPF₆, LiAsF₆, LiSbF₆, LiCF₃SO₃, LiBF₄, lithium bis(oxalato) borate, LiClO₄, LiN(SO₂C₂F₅)₂, LiN(SO₂CF₃)₂, LiN(SO₂F)₂, and LiPF₃(CF₂CF₃)₃, more preferred the conducting salt is selected from LiPF₆ and LiBF₄, and the most preferred conducting salt is LiPF₆.

The conducting salt(s) are usually present at a minimum concentration of at least 0.1 m/l, preferably the concentration of the conducting salt(s) is 0.5 to 2 mol/l based on the entire electrolyte composition.

Furthermore, electrolyte composition may contain at least one further additive different from the compounds of formula (I). The at least one further additive different from the compounds of formula (I) may be selected from polymers, film forming additives, flame retardants, overcharging additives, wetting agents, HF and/or H₂O scavenger, stabilizer for LiPF₆ salt, ionic solvation enhancer, corrosion inhibitors, and gelling agents.

The minimum concentration of the at least one further additive is usually 0.005 wt.-%, preferably the minimum concentration is 0.01 wt.-% and more preferred the minimum concentration is 0.1 wt.-%, based on the total weight of electrolyte composition. The maximum concentration of the at least further additive is usually 25 wt.-%.

One class of further additives are polymers. Polymers may be selected from polyvinylidene fluoride, polyvinylidene-hexafluoropropylene copolymers, polyvinylidene-hexafluoropropylene-chlorotrifluoroethylene copolymers, Nafion, polyethylene oxide, polymethyl methacrylate, polyacrylonitrile, polypropylene, polystyrene, polybutadiene, polyethylene glycol, polyvinylpyrrolidone, polyaniline, polypyrrole and/or polythiophene. Polymers may be added to a formulation according to the present invention in order to convert liquid formulations into quasi-solid or solid electrolytes and thus to improve solvent retention, especially during ageing. In this case they function as gelling agents.

Examples of flame retardants are organic phosphorous compounds like cyclophosphazenes, phosphoramides, alkyl and/or aryl tri-substituted phosphates, alkyl and/or aryl di- or tri-substituted phosphites, alkyl and/or aryl di- substituted phosphonates, alkyl and/or aryl tri-substituted phosphines, and fluorinated derivatives thereof.

Examples of HF and/or H₂O scavenger are optionally halogenated cyclic and acyclic silylamines.

Examples of overcharge protection additives are cyclohexylbenzene, o-terphenyl, p-terphenyl, and biphenyl and the like, preferred are cyclohexylbenzene and biphenyl.

Another class of additives are film forming additives, also called SEI-forming additives. An SEI forming additive according to the present invention is a compound which decomposes on an electrode to form a passivation layer on the electrode which prevents degradation of the electrolyte and/or the electrode. In this way, the lifetime of a battery is significantly extended. Preferably the SEI forming additive forms a passivation layer on the anode. An anode in the context of the present invention is understood as the negative electrode of a battery. Preferably, the anode has a reduction potential of 1 Volt or less against lithium such as a lithium intercalating graphite anode. In order to determine if a compound qualifies as anode film forming additive, an electrochemical cell can be prepared comprising a graphite electrode and a metal counter electrode, and an electrolyte containing a small amount of said compound, typically from 0.1 to 10 wt.-% of the electrolyte composition, preferably from 0.2 to 5 wt.-% of the electrolyte composition. Upon application of a voltage between anode and lithium metal, the differential capacity of the electrochemical cell is recorded between 0.5 V and 2 V. If a significant differential capacity is observed during the first cycle, for example -150 mAh/V at 1 V, but not or essentially not during any of the following cycles in said voltage range, the compound can be regarded as SEI forming additive.

According to the present invention the electrolyte composition preferably contains at least one SEI forming additive. SEI forming additives are known to the person skilled in the art. More preferred the electrolyte composition contains at least one SEI forming selected from vinylene carbonate and its derivatives such as vinylene carbonate and methylvinylene carbonate; fluorinated ethylene carbonate and its derivatives such as monofluoroethylene carbonate, cis- and trans-difluorocarbonate; organic sultones such as propylene sultone, propane sultone and their derivatives; ethylene sulfite and its derivatives; oxalate comprising compounds such as lithium oxalate, oxalato borates including dimethyl oxalate, lithium bis(oxalate) borate, lithium difluoro (oxalato) borate, and ammonium bis(oxalato) borate, and oxalato phosphates including lithium tetrafluoro (oxalato) phosphate; and ionic compounds containing a cation of formula (II) 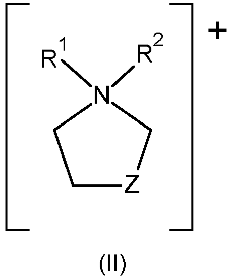 wherein
Z is CH₂ or NR¹³,
R¹ is selected from C₁ to C₆ alkyl,
R² is selected from -(CH₂)ᵤ-SO₃-(CH₂)ᵥ-R¹⁴, -SO₃- is -O-S(O)₂- or -S(O)₂-O-, preferably -SO₃- is -O-S(O)₂-,
u is an integer from 1 to 8, preferably u is 2, 3 or 4, wherein one or more CH₂ groups of the - (CH₂)ᵤ- alkylene chain which are not directly bound to the N-atom and/or the SO₃ group may be replaced by O and wherein two adjacent CH₂ groups of the -(CH₂)ᵤ- alkylene chain may be replaced by a C-C double bond, preferably the -(CH₂)ᵤ- alkylene chain is not substituted and u is an integer from 1 to 8, preferably u is 2, 3 or 4,
v is an integer from 1 to 4, preferably v is 0,
R¹³ is selected from C₁ to C₆ alkyl,
R¹⁴ is selected from C₁-C₂₀ alkyl, C₂-C₂₀ alkenyl, C₂-C₂₀ alkynyl, C₆-C₁₂ aryl, and C₆-C₂₄ aralkyl, which may contain one or more F, and wherein one or more CH₂ groups of alkyl, alkenyl, alkynyl and aralkyl which are not directly bound to the SO₃ group may be replaced by O, preferably R¹⁴ is selected from C₁-C₆ alkyl, C₂-C₄ alkenyl, and C₂-C₄ alkynyl, which may contain one or more F, and wherein one or more CH₂ groups of alkyl, alkenyl, alkynyl and aralkyl which are not directly bound to the SO₃ group may be replaced by O, preferred examples of R¹⁴ include methyl, ethyl, trifluoromethyl, pentafluoroethyl, n-propyl, n-butyl, n-hexyl, ethenyl, ethynyl, allyl or prop-1-yn-yl,
and an anion selected from bisoxalato borate, difluoro (oxalato) borate, [F_{z}B(CₙF_{2y+1})_{4-z}]⁻, [F_{y}P(CₙF₂ₙ₊₁)_{6-y}]⁻, (CyF₂ₙ₊₁)₂P(O)O]⁻, [CyF₂ₙ₊₁P(O)O₂]²⁻, [O-C(O)-CₙF₂ₙ₊₁]⁻, [O-S(O)₂-CₙF₂ₙ₊₁]⁻, [N(C(O)-CₙF₂ₙ₊₁)₂]⁻, [N(S(O)₂-CₙF₂ₙ₊₁)₂]⁻, [N(C(O)-CₙF₂ₙ₊₁)(S(O)₂-CₙF₂ₙ₊₁)]⁻, [N(C(O)-CₙF₂ₙ₊₁)(C(O)F)]⁻, [N(S(O)₂-CₙF₂ₙ₊₁)(S(O)₂F)]⁻, [N(S(O)₂F)₂]⁻, [C(C(O)-CₙF₂ₙ₊₁)₃]⁻, [C(S(O)₂-CₙF₂ₙ₊₁)₃]⁻, wherein n is an integer from 1 to 20, preferably up to 8, z is an integer from 1 to 4, and y is an integer from 1 to 6,

Preferred anions are bisoxalato borate, difluoro (oxalato) borate, [F₃B(CF₃)]⁻, [F₃B(C₂F₅)]⁻, [PF₆]⁻, [F₃P(C₂F₅)₃]⁻, [F₃P(C₃F₇)₃]⁻, [F₃P(C₄F₉)₃]⁻, [F₄P(C₂F₅)₂]⁻, [F₄P(C₃F₇)₂]⁻, [F₄P(C₄F₉)₂]⁻, [F₅P(C₂F₅)]⁻, [F₅P(C₃F₇)]⁻ or [F₅P(C₄F₉)]⁻, [(C₂F₅)₂P(O)O]⁻, [(C₃F₇)₂P(O)O]⁻ or [(C₄F₉)₂P(O)O]⁻. [C₂F₅P(O)O₂]²⁻, [C₃F₇P(O)O₂]²⁻, [C₄F₉P(O)O₂]²⁻, [O-C(O)CF₃]⁻, [O-C(O)C₂F₅]⁻, [O-C(O)C₄F₉]⁻, [O-S(O)₂CF₃]⁻, [O-S(O)₂C₂F₅]⁻, [N(C(O)C₂F₅)₂]⁻, [N(C(O)(CF₃)₂]⁻, [N(S(O)₂CF₃)₂]⁻, [N(S(O)₂C₂F₅)₂]⁻, [N(S(O)₂C₃F₇)₂]⁻, [N(S(O)₂CF₃)(S(O)₂C₂F₅)]⁻, [N(S(O)₂C₄F₉)₂]⁻, [N(C(O)CF₃)(S(O)₂CF₃)]⁻, [N(C(O)C₂F₅)(S(O)₂CF₃)]⁻ or [N(C(O)CF₃)(S(O)₂-C₄F₉)]⁻, [N(C(O)CF₃)(C(O)F)]⁻, [N(C(O)C₂F₅)(C(O)F)]⁻, [N(C(O)C₃F₇)(C(O)F)]⁻, [N(S(O)₂CF₃)(S(O)₂F)]⁻, [N(S(O)₂C₂F₅)(S(O)₂F)]⁻, [N(S(O)₂C₄F₉)(S(O)₂F)]⁻, [C(C(O)CF₃)₃]⁻, [C(C(O)C₂F₅)₃]⁻ or [C(C(O)C₃F₇)₃]⁻, [C(S(O)₂CF₃)₃]⁻, [C(S(O)₂C₂F₅)₃]⁻, and [C(S(O)₂C₄F₉)₃]⁻.

More preferred the anion is selected from bisoxalato borate, difluoro (oxalato) borate, CF₃SO₃⁻, and [PF₃(C₂F₅)₃]⁻.

The term "C₂-C₂₀ alkenyl" as used herein refers to an unsaturated straight or branched hydrocarbon group with 2 to 20 carbon atoms having one free valence. Unsaturated means that the alkenyl group contains at least one C-C double bond. C₂-C₆ alkenyl includes for example ethenyl, 1-propenyl, 2-propenyl, 1-n-butenyl, 2-n-butenyl, iso-butenyl, 1-pentenyl, 1-hexenyl, 1-heptenyl, 1-octenyl, 1-nonenyl, 1-decenyl and the like. Preferred are C₂-C₁₀ alkenyl groups, more preferred are C₂-C₆ alkenyl groups, even more preferred are C₂-C₄ alkenyl groups and in particular ethenyl and 1-propen-3-yl (allyl).

The term "C₂-C₂₀ alkynyl" as used herein refers to an unsaturated straight or branched hydrocarbon group with 2 to 20 carbon atoms having one free valence, wherein the hydrocarbon group contains at least one C-C triple bond. C₂-C₆ alkynyl includes for example ethynyl, 1-propynyl, 2-propynyl, 1-n-butinyl, 2-n-butynyl, iso-butinyl, 1-pentynyl, 1-hexynyl, - heptynyl, 1-octynyl, 1-nonynyl, 1-decynyl and the like and the like. Preferred are C₂-C₁₀ alkynyl, more preferred are C₂-C₆ alkynyl, even more preferred are C₂-C₄ alkynyl, in particular preferred are ethynyl and 1-propyn-3-yl (propargyl).

The term "C₆-C₁₂ aryl" as used herein denotes an aromatic 6- to 12-membered hydrocarbon cycle or condensed cycles having one free valence. Examples of C₆-C₁₂ aryl are phenyl and naphtyl. Preferred is phenyl.

The term "C₇-C₂₄ aralkyl" as used herein denotes an aromatic 6- to 12-membered aromatic hydrocarbon cycle or condensed aromatic cycles substituted by one or more C₁-C₆ alkyl. The C₇-C₂₄ aralkyl group contains in total 7 to 24 C-atoms and has one free valence. The free valence may be located at the aromatic cycle or at a C₁-C₆ alkyl group, i.e. C₇-C₂₄ aralkyl group may be bound via the aromatic part or via the alkyl part of the aralkyl group. Examples of C₇-C₂₄ aralkyl are methylphenyl, benzyl, 1,2-dimethylphenyl, 1,3-dimethylphenyl, 1,4-dimethylphenyl, ethylphenyl, 2-propylphenyl, and the like.

Compounds of formula (II) and their preparation are described in detail in WO 2013/026854 A1. Examples of compounds of formula (II) which are preferred according to the present invention are disclosed on page 12, line 21 to page 15, line 13 of WO 2013/026854 A1.

The electrolyte composition may contain 0.01 to 10 wt.-% of at least one further additive selected from organic carbonates having at least one C-C unsaturated bond, fluorinated organic carbonates, and inorganic fluoride salts. Preferably the at least one further additive is selected from organic cyclic carbonates having at least one C-C unsaturated bond, fluorinated organic cyclic carbonates, and LiPO₂F₂ The electrolyte composition may contain one of these compounds or mixtures of these compounds.

Organic carbonates containing at least one C-C unsaturated bond include cyclic carbonates and linear carbonates containing at least one C-C unsaturated bond. Organic cyclic carbonates containing at least one C-C unsaturated bond include cyclic carbonates wherein a double bond is part of the cycle like vinylene carbonate, methyl vinylene carbonate, and 4,5-dimethyl vinylene carbonate; and cyclic carbonate wherein the double bond is not part of the cycle, e.g. methylene ethylene carbonate, 4,5-dimethylene ethylene carbonate, vinyl ethylene carbonate, and 4,5-divinyl ethylene carbonate. Preferred cyclic carbonates containing at least one C-C unsaturated bond are vinylene carbonate, methyl vinylene carbonate, 4,5-dimethyl vinylene carbonate, methylene ethylene carbonate, and 4,5-dimethylene ethylene carbonate and more preferred vinylene carbonate. Examples of organic linear carbonates containing at least one C-C unsaturated bond are allylmethyl carbonate and diallyl carbonate.

The term "fluorinated organic cyclic carbonate(s)" means any cyclic organic carbonate as described above which is substituted by one or more F and includes fluorinated cyclic carbonates like monofluoroethylene carbonate (FEC), 4-fluoro-5-methyl ethylene carbonate, 4-(fluoromethyl) ethylene carbonate, 4-(trifluoromethyl) ethylene carbonate, and 4,5-difluoroethylene carbonate. A preferred fluorinated organic cyclic carbonate is monofluoroethylene carbonate.

The term "inorganic fluoride salts" means inorganic salts comprising an anion selected from fluorinated anions, e.g. [BF₄]⁻, bis(trifluormethylsulfonyl)imide anion, and [PO₂F₂]. The cation is preferably Li⁺. Examples of such inorganic fluoride salts are LiPO₂F₂, lithium bis(trifluormethylsulfonyl)imid, and LiBF₄. Preferred inorganic fluoride salts are LiBF₄ and LiPO₂F₂.

A compound added may have more than one effect in the electrolyte composition and the electrochemical cell comprising the electrolyte composition. E.g. lithium oxalato borate may be added as additive enhancing the SEI formation but can also function as conducting salt.

The electrolyte composition is preferably non-aqueous. In one embodiment of the present invention, the water content of the electrolyte composition is preferably below 100 ppm, based on the weight of the respective inventive formulation, more preferred below 50 ppm, most preferred below 30 ppm. The water content may be determined by titration according to Karl Fischer, e.g. described in detail in DIN 51777 or ISO760: 1978. The minimum water content of electrolyte compositions may be selected from 3 ppm, preferably 5 ppm.

In one embodiment of the present invention, the HF-content of the electrolyte composition is preferably below 100 ppm, based on the weight of the respective inventive formulation, more preferred below 50 ppm, most preferred below 30 ppm. The minimum HF content of inventive formulations may be selected from 5 ppm, preferably 10 ppm. The HF content may be determined by titration.

The electrolyte composition is preferably liquid at working conditions; more preferred it is liquid at 1 bar and 25 °C, even more preferred the electrolyte composition is liquid at 1 bar and -15 °C, in particular the electrolyte composition is liquid at 1 bar and -30 °C, even more preferred the electrolyte composition is liquid at 1 bar and -50 °C. Such liquid electrolyte compositions are particularly suitable for outdoor applications, for example for use in automotive batteries.

The electrolyte composition may be prepared by methods which are known to the person skilled in the field of the production of electrolytes, generally by dissolving a conductive salt in the corresponding solvent or solvent mixture and adding the at least one compound of formula (I) and optionally further additive(s), as described above.

In a further aspect the present invention as defined in claim 14 relates to an electrochemical cell comprising an electrolyte composition as described above or described as being preferred.

The electrochemical cell comprises
(A) an anode comprising at least one anode active material;
(B) a cathode comprising at least one cathode active material; and
(C) an electrolyte composition as described above or as described as being preferred.

The electrochemical cell may be a lithium battery, a double layer capacitor, or a lithium ion capacitor. The general construction of such electrochemical devices is known and is familiar to the person skilled in this art - for batteries, for example, in Linden's Handbook of Batteries (ISBN 978-0-07-162421-3).

Preferably the electrochemical cell is a lithium battery. The term "lithium battery" as used herein means an electrochemical cell, wherein the anode comprises lithium metal or lithium ions sometime during the charge/discharge of the cell. The anode may comprise lithium metal or a lithium metal alloy, a material occluding and releasing lithium ions, or other lithium containing compounds; e.g. the lithium battery may be a lithium ion battery, a lithium/sulphur battery, or a lithium/selenium sulphur battery. The lithium battery is preferably a secondary lithium battery, i.e. a rechargeable lithium battery.

In particular preferred embodiments the electrochemical cell is a lithium ion battery, i.e. a secondary lithium ion electrochemical cell comprising a cathode (A) comprising a cathode active material that can reversibly occlude and release lithium ions and an anode (B) comprising an anode active material that can reversibly occlude and release lithium ions.

Anode (A) comprises an anode active material that can reversibly occlude and release lithium ions or is capable to form an alloy with lithium. In particular carbonaceous material that can reversibly occlude and release lithium ions can be used as anode active material. Carbonaceous materials suited are crystalline carbon such as a graphite materials, more particularly, natural graphite, graphitized cokes, graphitized MCMB, and graphitized MPCF; amorphous carbon such as coke, mesocarbon microbeads (MCMB) fired below 1500°C, and mesophase pitch-based carbon fiber (MPCF); hard carbon; and carbonic anode active material (thermally decomposed carbon, coke, graphite) such as a carbon composite, combusted organic polymer, and carbon fiber.

Further examples of anode active materials are lithium metal and materials containing an element capable of forming an alloy with lithium. Non-limiting examples of materials containing an element capable of forming an alloy with lithium include a metal, a semimetal, or an alloy thereof. It should be understood that the term "alloy" as used herein refers to both alloys of two or more metals as well as alloys of one or more metals together with one or more semimetals. If an alloy has metallic properties as a whole, the alloy may contain a nonmetal element. In the texture of the alloy, a solid solution, a eutectic (eutectic mixture), an intermetallic compound or two or more thereof coexist. Examples of such metal or semimetal elements include, without being limited to, titanium (Ti), tin (Sn), lead (Pb), aluminum, indium (In), zinc (Zn), antimony (Sb), bismuth (Bi), gallium (Ga), germanium (Ge), arsenic (As), silver (Ag), hafnium (Hf), zirconium (Zr) yttrium (Y), and silicon (Si). Metal and semimetal elements of Group 4 or 14 in the long-form periodic table of the elements are preferable, and especially preferable are titanium, silicon and tin, in particular silicon. Examples of tin alloys include ones having, as a second constituent element other than tin, one or more elements selected from the group consisting of silicon, magnesium (Mg), nickel, copper, iron, cobalt, manganese, zinc, indium, silver, titanium (Ti), germanium, bismuth, antimony and chromium (Cr). Examples of silicon alloys include ones having, as a second constituent element other than silicon, one or more elements selected from the group consisting of tin, magnesium, nickel, copper, iron, cobalt, manganese, zinc, indium, silver, titanium, germanium, bismuth, antimony and chromium.

Further possible anode active materials are silicon based materials. Silicon based materials include silicon itself, e.g. amorphous and crystalline silicon, silicon containing compounds, e.g. SiOₓ with 0 < x < 1.5 and Si alloys, and compositions containing silicon and/or silicon containing compounds, e.g. silicon/graphite composites and carbon coated silicon containing materials. Silicon itself may be used in different forms, e.g. in the form of nanowires, nanotubes, nanoparticles, films, nanoporous silicon or silicon nanotubes. The silicon may be deposited on a current collector. Current collector may be selected from coated metal wires, a coated metal grid, a coated metal web, a coated metal sheet, a coated metal foil or a coated metal plate. Preferably, current collector is a coated metal foil, e.g. a coated copper foil. Thin films of silicon may be deposited on metal foils by any technique known to the person skilled in the art, e.g. by sputtering techniques. One method of preparing thin silicon film electrodes are described in R. Elazari et al.; Electrochem. Comm. 2012, 14, 21-24.

Other possible anode active materials are lithium ion intercalating oxides of Ti.

Preferably the anode active material comprises carbonaceous material that can reversibly occlude and release lithium ions, particularly preferred the carbonaceous material that can reversibly occlude and release lithium ions is selected from crystalline carbon, hard carbon and amorphous carbon, and particularly preferred is graphite. It is also preferred that the anode active material comprises silicon based anode active materials. It is further preferred that the anode active material comprises lithium ion intercalating oxides of Ti.

The inventive electrochemical cell comprises a cathode (B) comprising at least one cathode active material. The at least one cathode active material preferably comprises a material capable of occluding and releasing lithium ions selected from lithiated transition metal phosphates and lithium ion intercalating metal oxides.

Examples of lithium ion intercalating metal oxides are transition metal oxides like LiCoO₂, LiMnO₂, and lithium transition metal oxides with layered structure having the general formula Li_{(1+y)}[NiₐCo_{b}Mn_{c}]_{(1-y)}O₂₊ₑ wherein y is 0 to 0.3; a, b and c may be same or different and are independently 0 to 0.8 wherein a + b + c = 1; and -0.1 ≤ e ≤ 0. Another example of lithium transition metal oxides with layered structure are lithium intercalating mixed oxides of Ni, Co and Al. Preferred lithium intercalating mixed oxides of Ni, Co and Al have the general formula Li[NiₕCoᵢAlⱼ]O₂ wherein h is 0.7 to 0.9, preferred 0.8 to 0.87, and more preferred 0.8 to 0.85; i is 0.15 to 0.20; and j is 0.02 to 10, preferred 0.02 to 1, more preferred 0.02 to 0.1, and most preferred 0.02 to 0.03.

Further examples of lithium transition metal oxides are manganese-containing spinels are compounds of general formula Li₁₊ₜM₂₋ₜO_{4-d} wherein d is 0 to 0.4, t is 0 to 0.4 and M is Mn and at least one further metal selected from Co and Ni.

Examples of lithiated transition metal phosphates are LiMnPO₄, LiFePO₄ and LiCoPO₄.

In a preferred embodiment of the present invention cathode (B) contains at least one cathode active material selected from lithium intercalating mixed oxides of Ni, Co and Al, and lithium transition metal oxides with layered structure containing Ni, Co and Mn as described above, preferred lithium transition metal oxides with layered structure containing Ni, Co and Mn are those wherein the molar ratio of Ni: (Co + Mn) is at least 1:1, in particular preferred are Li[Ni_{0.8}Co_{0.1}Mn_{0.1}]O₂ (NCM 811), Li[Ni_{0.6}CO_{0.2}Mn_{0.2}]O₂ (NCM 622), and Li[Ni_{0.5}Co_{0.2}Mn_{0.3}]O₂ (NCM 523).

Cathode (B) may contain further components like binders and electrically conductive materials such as electrically conductive carbon. For example, cathode (B) may comprise carbon in a conductive polymorph, for example selected from graphite, carbon black, carbon nanotubes, graphene or mixtures of at least two of the aforementioned substances. Examples of binders used in cathode (B) are organic polymers like polyethylene, polyacrylonitrile, polybutadiene, polypropylene, polystyrene, polyacrylates, polyvinyl alcohol, polyisoprene and copolymers of at least two comonomers selected from ethylene, propylene, styrene, (meth)acrylonitrile and 1,3-butadiene, especially styrene-butadiene copolymers, and halogenated (co)polymers like polyvinlyidene chloride, polyvinly chloride, polyvinyl fluoride, polyvinylidene fluoride (PVdF), polytetrafluoroethylene, copolymers of tetrafluoroethylene and hexafluoropropylene, copolymers of tetrafluoroethylene and vinylidene fluoride and polyacrylnitrile.

Anode (A) and cathode (B) may be made by preparing an electrode slurry composition by dispersing the electrode active material, a binder, optionally a conductive material and a thickener, if desired, in a solvent and coating the slurry composition onto a current collector. The current collector may be a metal wire, a metal grid, a metal web, a metal sheet, a metal foil or a metal plate. Preferred the current collector is a metal foil, e.g. a copper foil or aluminum foil.

The inventive electrochemical cells may contain further constituents customary per se, for example separators, housings, cable connections etc. The housing may be of any shape, for example cuboidal or in the shape of a cylinder, the shape of a prism or the housing used is a metal-plastic composite film processed as a pouch. Suited separators are for example glass fiber separators and polymer-based separators like polyolefin or Nafion separators.

Several inventive electrochemical cells may be combined with one another, for example in series connection or in parallel connection. Series connection is preferred. The present invention further provides for the use of inventive electrochemical cells as described above in devices, especially in mobile devices. Examples of mobile devices are vehicles, for example automobiles, bicycles, aircraft, or water vehicles such as boats or ships. Other examples of mobile devices are those which are portable, for example computers, especially laptops, telephones or electrical power tools, for example from the construction sector, especially drills, battery-driven screwdrivers or battery-driven staplers. But the inventive electrochemical cells can also be used for stationary energy stores.

The present invention is further illustrated by the following examples that do not, however, restrict the invention.

### 1) Synthesis of additives

### (1-1) Synthesis of 2-chloroethanesulfonyl fluoride:

2-Chloroethanesulfonyl chloride (500 g, 2910 mmol) was added to a saturated KHF₂ solution in water (1700 ml) cooled by an ice bath, and the two phase mixture was stirred for 15 h at room temperature. The reaction mixture was diluted with water, extracted with dichloromethane (DCM), and dried over anhydrous Na₂SO₄. The solvent was removed under reduced pressure to give the product (386 g, 86% yield).

### (1-2) Synthesis of 2-cyanoethanesulfonyl fluoride (method A):

Tetrabutylammonium fluoride trihydrate (29 g, 88 mmol, 1.1 eq) was added to a solution of 2-chloroethanesulfonyl fluoride prepared according to (1-1) (12 g, 80 mmol, 1.0 eq) in tetrahydrofurane (THF) (80 ml) cooled by an ice bath. The mixture was stirred for 30 min, and became a pale brown solution. Trimethylsilyl cyanide (11 ml, 88 mol, 1.1 eq) was added slowly, and the solution became dark brown. The reaction temperature was increased to 50 °C, and stirred for 15 h. The reaction mixture was quenched with water, extracted with ethyl acetate, washed with brine, and dried over anhydrous Na₂SO₄. The solvent was removed under reduced pressure and the crude product was purified by silica gel chromatography. The obtained oil was purified again by distillation to give the product as a color less oil (6.0 g, 50% yield).

### (1-3) Synthesis of 2-cyanoethanesulfonyl fluoride (method B):

MgO (31 g, 773 mmol, 0.54 eq) was added to a solution of 2-chloroethanesulfonyl fluoride prepared according to (1-1) (221 g, 1433 mmol, 1.0 eq) in H₂O/ethanol = 1/1 (1200 ml) cooled by an ice bath, the mixture was stirred for 15 min, and became a white suspension. KCN (100 g, 1504 mol, 1.05 eq) was added slowly, and the reaction mixture became a dark brown suspension. The reaction temperature was kept under 35 °C. The reaction mixture was stirred for 1 h in an ice bath. The reaction mixture was quenched with water, extracted with AcOEt, washed with brine, and dried over anhydrous Na₂SO₄. The solvent was removed under reduced pressure and the crude product was filtrated by short silica gel filter. The obtained oil was purified by distillation to give the product as a color less oil (130 g, 65% yield).

### (1-4) Synthesis of methanesulfonyl fluoride:

Methanesulfonyl chloride (115 g, 1.00 mol) was added to a saturated KHF₂ solution in water 300 ml) in an ice bath was added, and the two phase mixture was stirred for 15 h at room temperature. The reaction mixture was diluted with water, extracted by DCM, and dried over anhydrous Na₂SO₄. The solvent was removed under reduced pressure and the crude product was purified by distillation to give the product as a color less oil (65 g, 66% yield).

### (1-5) Synthesis of methanedisulfonyl fluoride:

KF (26 g, 440 mmol, 8 eq) and 18-crown-6-ether (14.8 g, 55 mmol, 1 eq) were added to a suspension of methanedisulfonyl chloride (12 g, 55 mmol, 1.0 eq) in acetonitrile (300 ml) in an ice bath, the mixture was stirred for 15 h at room temperature. The reaction mixture was diluted with water, extracted by AcOEt, washed with brine, and dried over anhydrous Na₂SO₄. The solvent was removed under reduced pressure and the crude product was purified by distillation to give the product as a color less oil (3.0 g, 45% yield).

### (1-6) Synthesis of ethanedisulfonyl fluoride:

Ethanedisulfonyl fluoride (13 g, 58 mmol) was added to a saturated KHF₂ solution in water (100 ml) cooled by an ice bath, and the two phase mixture was stirred for 15 h at room temperature. The reaction mixture was diluted with water, extracted by DCM, and dried over anhydrous Na₂SO₄. The solvent was removed under reduced pressure and the crude product was purified by distillation to give the product as a color less oil (2.6 g, 23% yield).

### (1-7) Synthesis of 3,3-dicyano-pentane-1,5-disulfonyl fluoride:

Tetrabutylammonium fluoride trihydrate (1.63 g, 5 mmol, 0.05 eq) was added to a solution of malononitrile (6.7 g, 100 mmol, 1.0 eq) and vinylsulfonyl fluoride (12.2 g, 100 mmol, 1.0 eq) in EtOH (500 ml) at room temperature, the mixture was stirred for 3 h, and became white suspension. The reaction mixture was quenched with water, extracted with AcOEt, washed with brine, and dried over anhydrous Na₂SO₄. The solvent was removed under reduced pressure and the crude product was purified by silica gel chromatography to give the product as a color less solid (5.8 g, 20% yield).

### 2) Electrolyte compositions

A base electrolyte composition was prepared containing 12.7 wt% of LiPF₆, 26.2 wt% of ethylene carbonate (EC), and 61.1 wt% of ethyl methyl carbonate (EMC) (EL base 1), based on the total weight of ELbase1. To this ELbase1 formulation 2 wt% VC (EL base 2), 2 wt% FEC (EL base 3) and 10 wt% FEC (EL base 4) were added. To these base electrolyte compositions different amounts of additives were added. Vinyl sulfonylfluoride and methyl fluorosulfonate were purchased, the other additives were synthetized as described above. The exact compositions are summarized in Tables 1 to 10. In the Tables concentrations are given as wt.-% based on the total weight of the electrolyte composition. The molar concentration of the different additives was the same.

### 3) Silicon suboxide/graphite anodes

Silicon suboxide, graphite and carbon black were thoroughly mixed. CMC (carboxymethyl cellulose) aqueous solution and SBR (styrene butadiene rubber) aqueous solution were used as binder. The mixture of silicon oxide, graphite and carbon black was mixed with the binder solutions and an adequate amount of water was added to prepare a suitable slurry for electrode preparation. The thus obtained slurry was coated by using a roll coater onto copper foil (thickness = 18 µm) and dried under ambient temperature. The sample loading for electrodes on Cu foil was fixed to be 5 mg cm⁻² for coin type cell testing and 7mg cm⁻² for NCA//silicon suboxide/graphite pouch cell (200mAh) testing.

### 4) Fabrication of cathode tapes

### (4-1) Fabrication of NCM523 cathode tapes

Lithium containing mixed Ni, Co and Mn oxide (NCM 523, manufactured by BASF) was used as a cathode active material and mixed with carbon black. The mixture of NCM 523 and carbon black was mixed with polyvinylidene fluoride (PVdF) binders, and an adequate amount of N-methylpyrrolidinone (NMP) was added to prepare a suitable slurry for electrode preparation. The thus obtained slurry was coated by using a roll coater onto aluminum foil (thickness = 15 µm) and dried under ambient temperature. This electrode tape was then kept at 130 °C under vacuum for 8 h to be ready to be used. The thickness of the cathode active material was found to be 72 µm, which was corresponding to 12.5 mg/cm² of the loading amount.

### (4-2) Fabrication of NCA cathode tapes

Lithium containing mixed Ni, Co and Al oxide (NCA) was used as a cathode active material. Method for fabricating the NCA tape is same as described above for NCM523. The thickness of the cathode active material was found to be 50 µm, which was corresponding to 11 mg/cm² of the loading amount.

### 5) Fabrication of the test cells

Coin-type full cells (20 mm in diameter and 3.2 mm in thickness) comprising a NCM 523 cathode prepared as described above under (4-1) and a silicon suboxide/graphite composite anode prepared as described above under 3) as cathode and anode electrode, respectively, were assembled and sealed in an Ar-filled glove box. In addition, the cathode and anode described above and a separator were superposed in order of cathode // separator // anode to produce a coin full cell. Thereafter, 0.15 mL of the different nonaqueous electrolyte compositions were introduced into the coin cell.

Pouch cells (350 mAh) comprising a NCM 523 electrode prepared as described above in (4-1) and a graphite electrode as cathode and anode, respectively, were assembled and sealed in an Ar-filled glove box. In addition, the cathode and anode described above and a separator were superposed in order of cathode // separator // anode to produce a several layers pouch cell. Thereafter, 3 mL of the different nonaqueous electrolyte compositions were introduced into the Laminate pouch cell.

Pouch cells (200 mAh) comprising a NCA electrode prepared as described above in (4-2) and a silicon suboxide/graphite electrode as cathode and anode, respectively, were assembled and sealed in an Ar-filled glove box. In addition, the cathode and anode described above and a separator were superposed in order of cathode // separator // anode to produce a several layers pouch cell. Thereafter, 0.7 mL of the different nonaqueous electrolyte compositions were introduced into the Laminate pouch cell.

### 6) Cycling stability of coin full cell comprising NCM523// silicon suboxide /graphite composite anode

Coin full cells prepared comprising a NCM523 cathode and silicon suboxide/graphite composite anode were tested in a voltage range between 4.2 V to 2.5 V at room temperature. For the initial 2 cycles, the initial charge was conducted in the CC-CV mode, i.e., a constant current (CC) of 0.05 C was applied until reaching 0.01 C. After 5 min resting time, discharge was carried out at constant current of 0.05 C to 2.5 V. For the cycling, the current density increased to 0.5 C. The results are summarized in Table 1, 2, 3 and 4. [%] Discharge capacity after 200 cycles is based on the capacity of EL base 2 cell after 200 cycles as 100%.

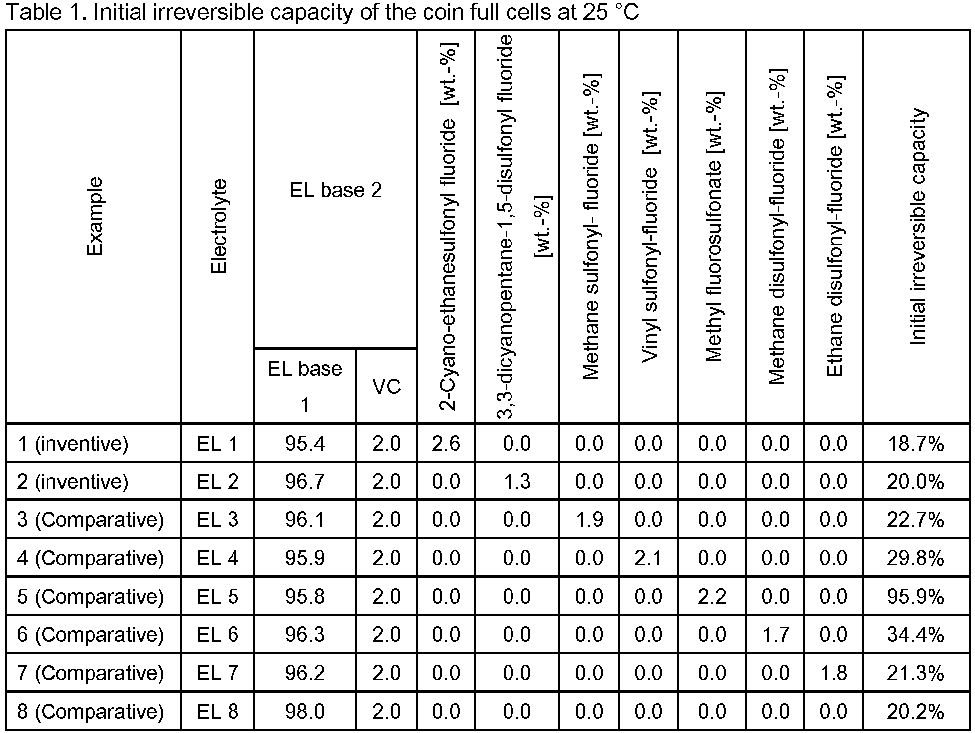

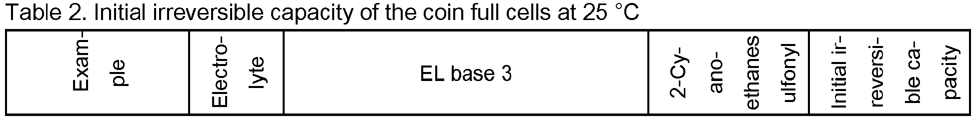

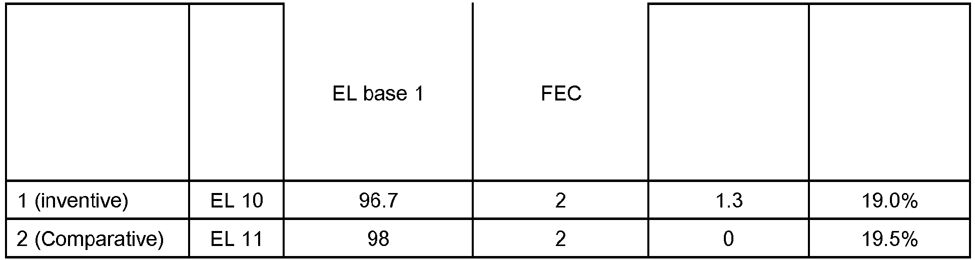

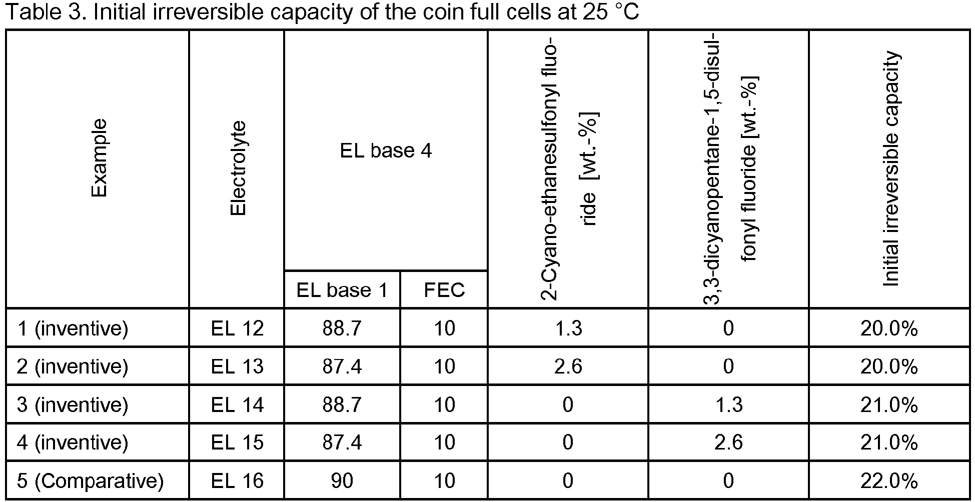

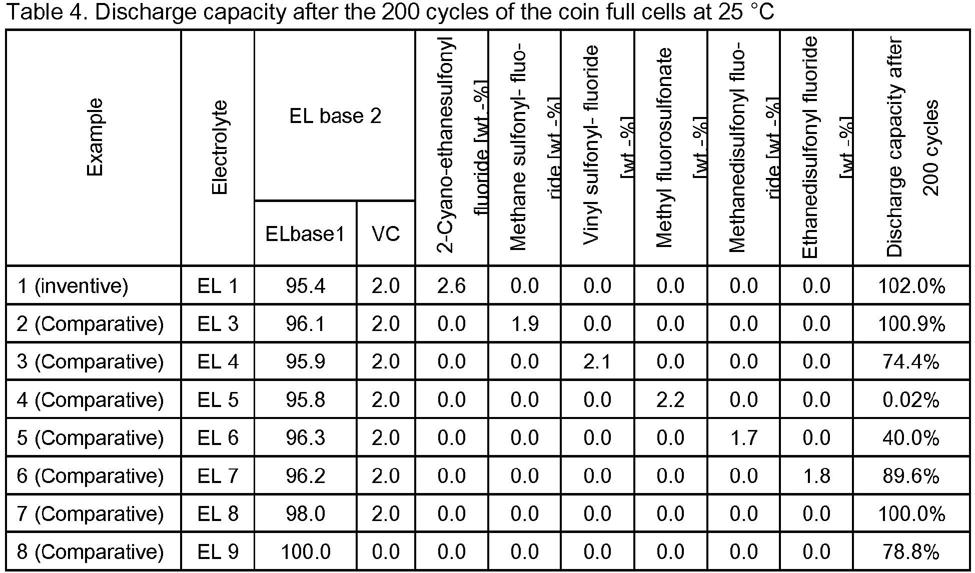

As can be seen from Tables 1, 2, 3 and 4, the inventive electrochemical cell shows the lowest initial irreversible capacity and shows the best discharge capacity after 200 charge/discharge cycles at 25 °C.

### 7) Evaluation of high-temperature storability of pouch cell comprising NCM523//graphite anode

### 7-1) AC impedance at 3.1 V and at 4.25 V

The pouch cells (350mAh) prepared comprising a NCM523 cathode and graphite anode were charged to 3.1 V at a constant current of 0.1 C and then charged at a constant voltage of 3.1 V until the current value reached 0.01 C at initial cycles. For these cells the AC impedance was measured at 25 °C. Afterwards, the cells were degassed. After degassing, the pouch cells were charged to 4.2 V at the constant current of 0.1 C and then they were charged at a constant voltage of 4.2 V until the current value reached 0.01 C. These cells were stored at 45 °C for 5 days and then transferred to 25 °C to check the retention capacity and recover capacity. After the capacity check, the pouch cells was charged to 4.25 V at a constant current of 0.1 C and then charged at a constant voltage of 4.25 V until the current value reached 0.01 C after the formation cycles. For these cells the AC impedance was measured at 4.25 V at 25 °C. The results are shown in Table 5.

### 7-2) Gas amount after storage at 60 °C for 30 days at 4.25 V

These cells was stored at 60 °C for 30 days and then cooled. The cells was measured by Archimedes method to identify the volume change before and after storage. The gas amount of the cells is determined as the ratio of the volume change before and after storage of cells and is given in % based on the gas amount of pouch cell with EL 9 electrolyte (EL base 1). The results are summarized in Table 6.

### 8) Evaluation of high-temperature storability of pouch full cell comprising NCA//Silicon suboxide /graphite composite anode

### (8-1) Gas amount after aging at 45 °C for 5days at 4.2V

The pouch cells (200mAh) prepared comprising a NCA cathode and silicon suboxide/graphite anode were charged to 3.1 V at a constant current of 0.1 C and then charged at a constant voltage of 3.1 V until the current value reached 0.01 C at initial cycles. Afterwards, these cells were degassed. After degassing, cell volume was measured by Archimedes method. After then, the pouch cells charged to 4.2 V at the constant current of 0.1 C and then charged at a constant voltage of 4.2 V until the current value reached 0.01 C. These cells were stored at 45 °C for 5 days and then transferred to 25 °C to check the retention capacity and recover capacity. After the capacity check, cell volume was measured again. The aging gas amount of the cells is determined as the volume difference before and after aging of cells and is given in % based on the gas amount of pouch cell without 2-cyanoethane sulfonyl fluoride. The results are displayed in Tables 7 and 8.

### (8-2) Gas amount after storage at 60 °C for 30 days at 4.2 V

After the above formation step, these cells were stored at 60 °C for 30 days and cell volume was measured again. The storage gas amount of the cells is determined as the volume difference after degas and after storage of cells and is given in % based on the gas amount of pouch cell without 2-cyanoethane sulfonyl fluoride. The results are displayed in Tables 9 and 10.

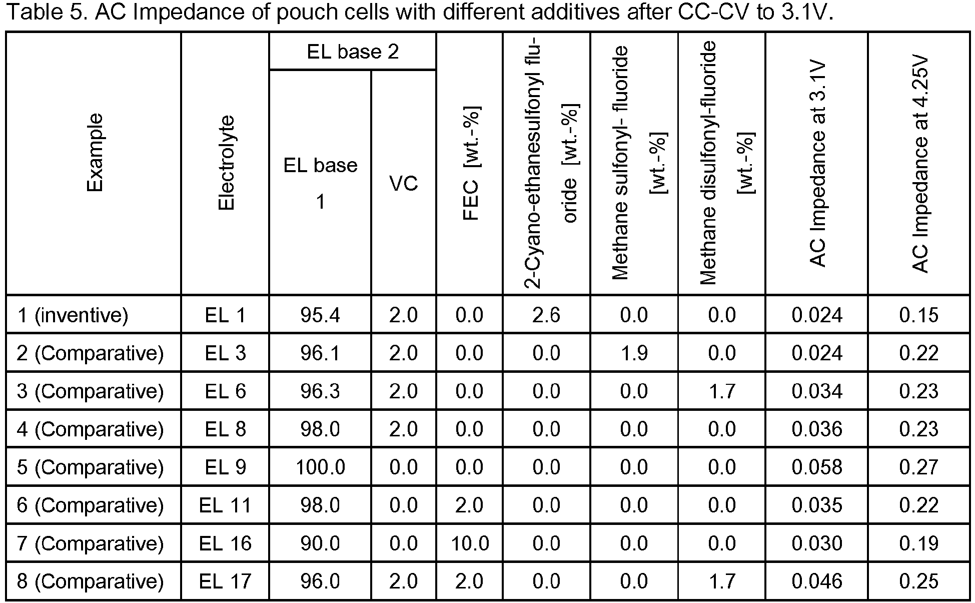

Table 5 shows the impedance for an inventive electrolyte composition containing 2-cyanoethane sulfonyl fluoride and for seven comparative electrolyte compositions. The addition of the 2-cyanoethane sulfonyl fluoride shows lowest AC resistance at 3.1V and also at 4.25V after the formation. It is considered that adding 2-cyanoethane sulfonyl fluoride to VC containing electrolyte could reduce charge transfer resistance.

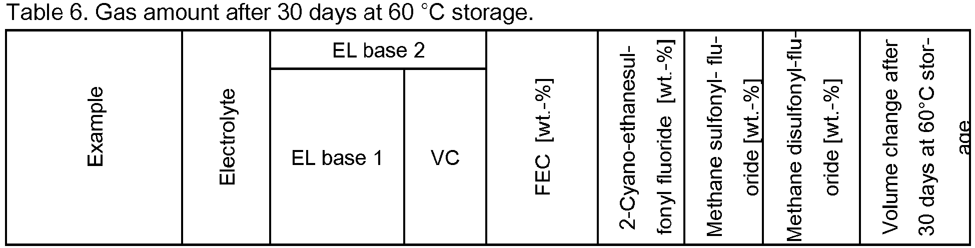

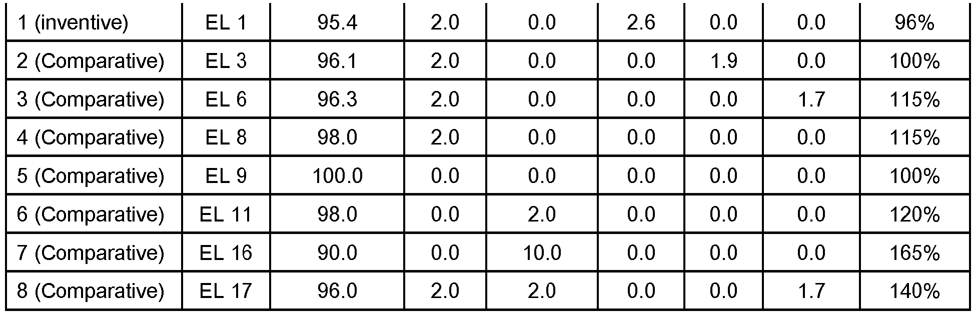

Table 6 shows that in the NCM523//graphite pouch cell, that by adding 2-cyanoethane sulfonyl fluoride to VC containing electrolyte the storage gas development is significantly reduced.

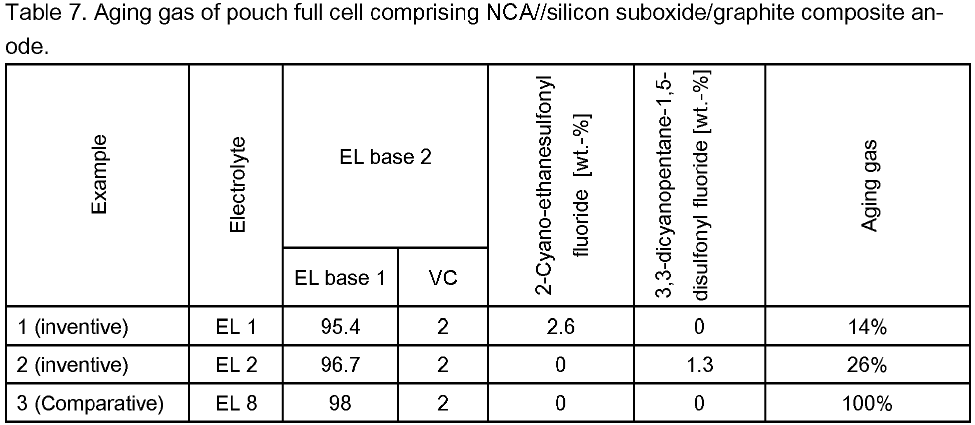

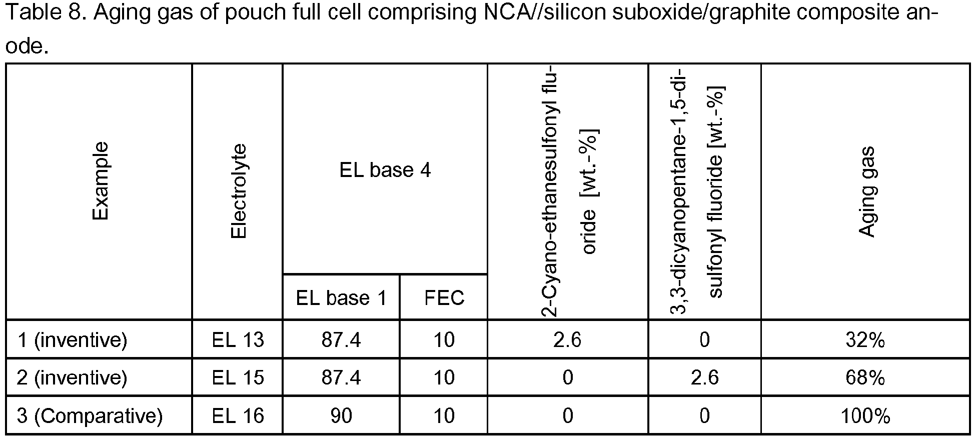

Tables 7 and 8 show that in the NCA//silicon suboxide/graphite pouch cell, the development of aging gas is significantly reduced by adding 2-cyanoethane sulfonyl fluoride and 3,3-dicyano-pentane-1,5-disulfonyl fluoride to a VC or FEC containing electrolyte.

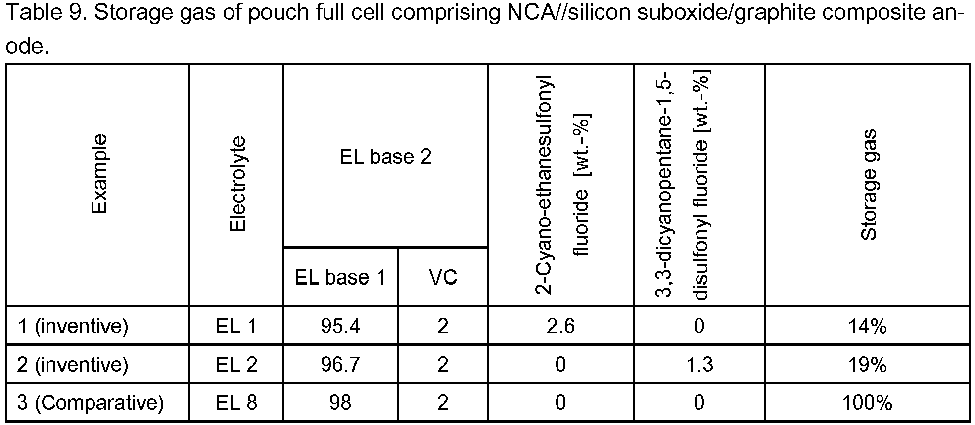

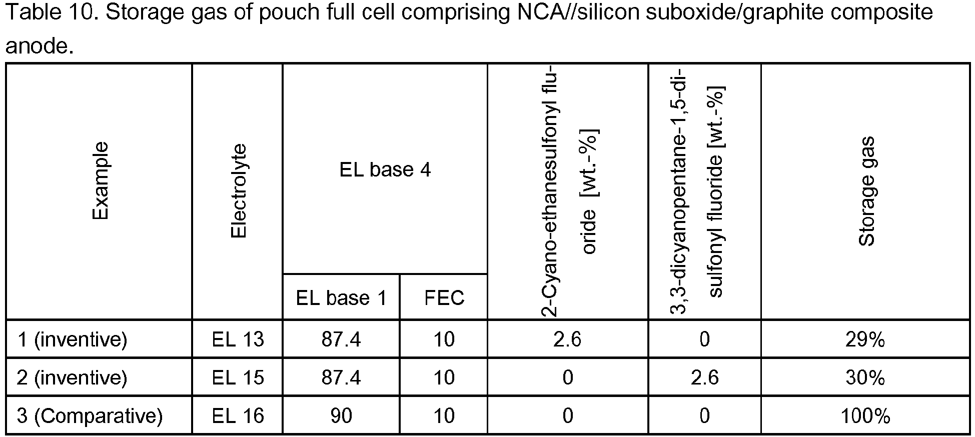

Tables 9 and 10 show that in the NCA//silicon suboxide/graphite pouch cell, the development of storage gas is significantly reduced by adding 2-cyanoethane sulfonyl fluoride and 3,3-dicyano-pentane-1,5-disulfonyl fluoride to a VC or FEC containing electrolyte.

## Claims

1. An electrolyte composition containing at least one compound of formula (I) 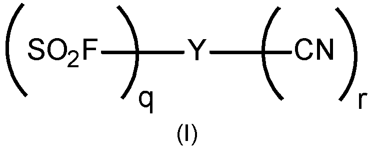 wherein
q is 1, 2 or 3,
r is 1, 2 or 3, and
Y is a q+r valent non-fluorinated alkylene group having 2 to 6 C-atoms.

2. The electrolyte composition according to claim 1, wherein r is 2 and the two sulfonylfluoride groups are bound to the alkylene group Y at terminal positions.

3. The electrolyte composition according to claim 1 or 2, wherein q is 2 and both CN-groups are bound to the same C-atom of the alkylene group Y.

4. The electrolyte composition according to any of claims 1 to 3, wherein q is 2 and r is 2.

5. The electrolyte composition according to claim 1, wherein q is 1 and r is 1.

6. The electrolyte composition according to any of claims 1 to 5, wherein compound of formula (I) is selected from 2-cyanoethanesulfonyl fluoride and 3,3-dicyano-pentane-1,5-disulfonyl fluoride.

7. The electrolyte composition according to any of claims 1 to 6, wherein the electrolyte composition contains in total 0.01 to 10 wt.-% of the at least one compound of formula (I), based on the total weight of the electrolyte composition.

8. The electrolyte composition according to any of claims 1 or 7, wherein the electrolyte composition contains at least one aprotic organic solvent selected from fluorinated and non-fluorinated cyclic and acyclic organic carbonates, di-C₁-C₁₀-alkylethers, di-C₁-C₄-alkyl-C₂-C₆-alkylene ethers and polyethers, cyclic ethers, cyclic and acyclic acetales and ketales, orthocarboxylic acids esters, cyclic and acyclic esters and diesters of carboxylic acids, cyclic and acyclic sulfones, and cyclic and acyclic nitriles and dinitriles and mixtures thereof.

9. The electrolyte composition according to any of claims 1 to 8, wherein the electrolyte composition contains at least one lithium conducting salt.

10. The electrolyte composition according to any of claims 1 to 9, wherein the electrolyte composition contains at least one conducting salt selected from LiPF₆, LiAsF₆, LiSbF₆, LiCF₃SO₃, LiBF₄, lithium bis(oxalato) borate, lithium difluoro(oxalato) borate, LiClO₄, LiN(SO₂C₂F₅)₂, LiN(SO₂CF₃)₂, LiN(SO₂F)₂, and LiPF₃(CF₂CF₃)₃.

11. The electrolyte composition according to any of claims 1 to 10, wherein the electrolyte composition contains at least one further additive different from the compounds of formula (I) selected from polymers, film forming additives, flame retardants, overcharging additives, wetting agents, HF and/or H₂O scavenger, stabilizer for LiPF₆ salt, ionic solvation enhancer, corrosion inhibitors, and gelling agents.

12. The electrolyte composition according to any of claims 1 to 11, wherein the electrolyte composition contains 0.01 to 10 wt.-% of at least one further additive selected from organic carbonates having at least one C-C unsaturated bond, fluorinated organic carbonates, and inorganic fluoride salts.

13. The use of compounds of formula (I) 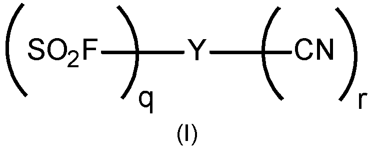 wherein
q is 1, 2 or 3,
r is 1, 2 or 3, and
Y is a q+r valent non-fluorinated alkylene group having 2 to 6 C-atoms,
in electrolyte compositions.

14. An electrochemical cell comprising the electrolyte composition according to any of claims 1 to 12.

## Patentansprüche

1. Elektrolytzusammensetzung, enthaltend mindestens eine Verbindung der Formel (I) 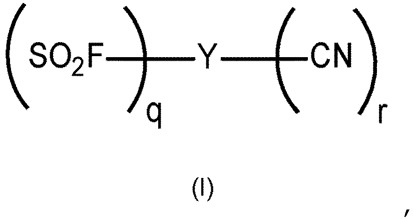 worin
q 1, 2 oder 3 ist,
r 1, 2 oder 3 ist und
Y für eine nichtfluorierte Alkylengruppe mit einer Valenz von q+r und 2 bis 6 C-Atomen steht.

2. Elektrolytzusammensetzung nach Anspruch 1, wobei r 2 ist und die beiden Sulfonylfluoridgruppen endständig an die Alkylengruppe Y gebunden sind.

3. Elektrolytzusammensetzung nach Anspruch 1 oder 2, wobei q 2 ist und beide CN-Gruppen an das gleiche C-Atom der Alkylengruppe Y gebunden sind.

4. Elektrolytzusammensetzung nach einem der Ansprüche 1 bis 3, wobei q 2 ist und r 2 ist.

5. Elektrolytzusammensetzung nach Anspruch 1, wobei q 1 ist und r 1 ist.

6. Elektrolytzusammensetzung nach einem der Ansprüche 1 bis 5, wobei die Verbindung der Formel (I) aus 2-Cyanoethansulfonylfluorid und 3,3-Dicyanopentan-1,5-disulfonylfluorid ausgewählt ist.

7. Elektrolytzusammensetzung nach einem der Ansprüche 1 bis 6, wobei die Elektrolytzusammensetzung insgesamt 0,01 bis 10 Gew.-%, bezogen auf das Gesamtgewicht der Elektrolytzusammensetzung, der mindestens einen Verbindung der Formel (I) enthält.

8. Elektrolytzusammensetzung nach einem der Ansprüche 1 oder 7, wobei die Elektrolytzusammensetzung mindestens ein aprotisches organisches Lösungsmittel enthält, das aus fluorierten und nichtfluorierten cyclischen und acyclischen organischen Carbonaten, Di-C₁-C₁₀-alkylethern, Di-C₁-C₄-alkyl-C₂-C₆-alkylenethern und Polyethern, cyclischen Ethern, cyclischen und acyclischen Acetalen und Ketalen, Orthocarbonsäureestern, cyclischen und acyclischen Estern und Diestern von Carbonsäuren, cyclischen und acyclischen Sulfonen und cyclischen und acyclischen Nitrilen und Dinitrilen und Mischungen davon ausgewählt ist.

9. Elektrolytzusammensetzung nach einem der Ansprüche 1 bis 8, wobei die Elektrolytzusammensetzung mindestens ein Lithium-Leitsalz enthält.

10. Elektrolytzusammensetzung nach einem der Ansprüche 1 bis 9, wobei die Elektrolytzusammensetzung mindestens ein Leitsalz, das aus LiPF₆, LiAsF₆, LiSbF₆, LiCF₃SO₃, LiBF₄, Lithiumbis(oxalato)borat,
Lithiumdifluoro(oxalato)borat, LiClO₄, LiN(SO₂C₂F₅)₂, LiN(SO₂CF₃)₂, LiN(SO₂F)₂ und LiPF₃(CF₂CF₃)₃ ausgewählt ist, enthält.

11. Elektrolytzusammensetzung nach einem der Ansprüche 1 bis 10, wobei die Elektrolytzusammensetzung mindestens ein weiteres Additiv enthält, das von den Verbindungen der Formel (I) verschieden ist und aus Polymeren, filmbildenden Additiven, Flammschutzmitteln, Überladeadditiven, Netzmitteln, HF- und/oder H₂O-Fänger, Stabilisator für LiPF₆-Salz, Mittel zur Verbesserung der Ionensolvatation, Korrosionsinhibitoren und Gelbildnern ausgewählt ist.

12. Elektrolytzusammensetzung nach einem der Ansprüche 1 bis 11, wobei die Elektrolytzusammensetzung 0,01 bis 10 Gew.-% mindestens eines weiteren Additivs, das aus organischen Carbonaten mit mindestens einer ungesättigten C-C-Bindung, fluorierten organischen Carbonaten und anorganischen Fluoridsalzen ausgewählt ist, enthält.

13. Verwendung von Verbindungen der Formel (I) 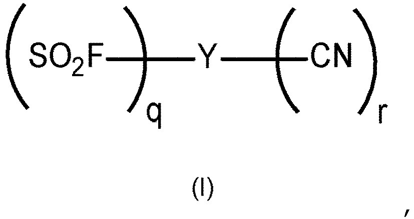 worin
q 1, 2 oder 3 ist,
r 1, 2 oder 3 ist und
Y für eine nichtfluorierte Alkylengruppe mit einer Valenz von q+r und 2 bis 6 C-Atomen steht,
in Elektrolytzusammensetzungen.

14. Elektrochemische Zelle, umfassend die Elektrolytzusammensetzung nach einem der Ansprüche 1 bis 12.

## Revendications

1. Composition d'électrolyte contenant au moins un composé de formule (I) 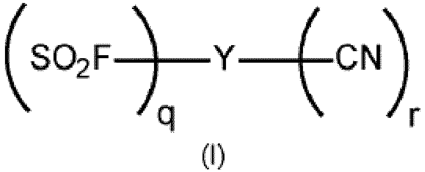
q étant 1, 2 ou 3,
r étant 1, 2 ou 3, et
Y étant un groupe alkylène non fluoré de valence q +r possédant 2 à 6 atomes de C.

2. Composition d'électrolyte selon la revendication 1, r étant 2 et les deux groupes fluorosulfonyle étant liés au groupe alkylène Y au niveau de positions terminales.

3. Composition d'électrolyte selon la revendication 1 ou 2, q étant 2 et les deux groupes CN étant liés au même atome de C du groupe alkylène Y.

4. Composition d'électrolyte selon l'une quelconque des revendications 1 à 3, q étant 2 et r étant 2.

5. Composition d'électrolyte selon la revendication 1, q étant 1 et r étant 1.

6. Composition d'électrolyte selon l'une quelconque des revendications 1 à 5, le composé de formule (I) étant choisi parmi le fluorure de 2-cyanoéthanesulfonyle et le fluorure de 3,3-dicyano-pentane-1,5-disulfonyle.

7. Composition d'électrolyte selon l'une quelconque des revendications 1 à 6, la composition d'électrolyte contenant au total 0,01 à 10 % en poids de l'au moins un composé de formule (I) sur la base du poids total de la composition d'électrolyte.

8. Composition d'électrolyte selon l'une quelconque des revendications 1 et 7, la composition d'électrolyte contenant au moins un solvant organique aprotique choisi parmi des carbonates organiques, des di-C₁₋₁₀-alkyléthers, des di-C₁₋₄-alkyl-C₂-₆-alkylènéthers et des polyéthers fluorés et non fluorés, cycliques et acycliques, des éthers cycliques, des acétals et des cétals cycliques et acycliques, des esters d'acides orthocarboxyliques, des esters et des diesters cycliques et acycliques d'acides carboxyliques, des sulfones cycliques et acycliques, et des nitriles et dinitriles cycliques et acycliques et des mélanges correspondants.

9. Composition d'électrolyte selon l'une quelconque des revendications 1 à 8, la composition d'électrolyte contenant au moins un sel conducteur de lithium.

10. Composition d'électrolyte selon l'une quelconque des revendications 1 à 9, la composition d'électrolyte contenant au moins un sel conducteur choisi parmi LiPF₆, LiAsF₆, LiSbF₆, LiCF₃SO₃, LiBF₄, bis(oxalato)borate de lithium, difluoro(oxalato)borate de lithium, LiClO₄, LiN(SO₂C₂F₅)₂, LiN(SO₂CF₃)₂, LiN(SO₂F)₂, et LiPF₃(CF₂CF₃)₃.

11. Composition d'électrolyte selon l'une quelconque des revendications 1 à 10, la composition d'électrolyte contenant au moins un additif supplémentaire différent des composés de formule (I) choisi parmi les polymères, les additifs filmogènes, les agents ignifugeants, les additifs de surcharge, les agents mouillants, un capteur pour HF et/ou H₂O, un stabilisant pour le sel LiPF₆, un agent augmentant la solvatation ionique, les inhibiteurs de corrosion, et les agents gélifiants.

12. Composition d'électrolyte selon l'une quelconque des revendications 1 à 11, la composition d'électrolyte contenant 0,01 à 10 % en poids d'au moins un additif supplémentaire choisi parmi les carbonates organiques possédant au moins une liaison C-C insaturée, les carbonates organiques fluorés, et les sels de fluorure inorganiques.

13. Utilisation des composés de formule (I) 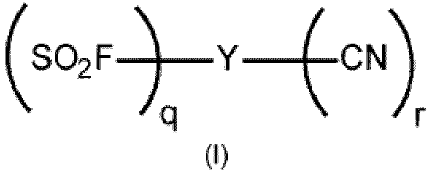
q étant 1, 2 ou 3,
r étant 1, 2 ou 3, et
Y étant un groupe alkylène non fluoré de valence q +r possédant 2 à 6 atomes de C,
dans des compositions d'électrolyte.

14. Cellule électrochimique comprenant la composition d'électrolyte selon l'une quelconque des revendications 1 à 12.
